# EUROPEAN PATENT SPECIFICATION

(11) **EP 0 489 750 B1**
(45) Date of publication and mention of the grant of the patent: **17.04.1996**
(21) Application number: 90911532.1
(22) Date of filing: 06.08.1990
(51) Int. Cl.: A61M 5/50

(54) **INSERTABLE ELEMENT FOR PREVENTING REUSE OF PLASTIC SYRINGES**
EINSATZELEMENT ZUM VERMEIDEN DER WIEDERVERWENDUNG VON SPRITZEN AUS KUNSTSTOFF
ELEMENT D'INTRODUCTION EMPECHANT LA REUTILISATION DE SERINGUES EN PLASTIQUE

(30) Priority: 07.08.1989 US 390083
(43) Date of publication of application: 17.06.1992
(73) Proprietor: ALLISON, Alan Chris, Pacifia, CA 94044 (US); JAFFE, Richard A., Palo Alto, CA 94303 (US)
(72) Inventor: ALLISON, Alan Chris, Pacifia, CA 94044 (US); JAFFE, Richard A., Palo Alto, CA 94303 (US)
(74) Representative: Vanderperre, Robert
(86) International application number: US9004406
(87) International publication number: WO9101768

(56) References cited:
- WO-A-89/02287
- US-A- 4 731 068

## Description

### Field of The Invention

This invention generally relates to a device for preventing reuse of a syringe, and more particularly, to one which can be retrofit to or inserted in standard plastic syringes.

### Background Art

The reuse of needle syringes for intravenous injections has been recognized as a cause of the spread of communicable diseases. Particularly with the current spread of AIDS, it has become desireable to discourage the reuse of unclean syringes. Disposable plastic syringes are widely available because of their low cost and convenience in handling. Although they are intended to be discarded after a single use, they are in fact commonly reused without proper disinfection, particularly in the case of intraveneous drug users.

Prior techniques for preventing reuse of needle syringes include various arrangements for locking out the plunger of a syringe after it has been once loaded and depressed to the end of its travel to inject the contents of the syringe. For example, U.S. Patent 4,731,068 to Hesse discloses a two-part lock construction having a band or sleeve assembled at the injection end of the syringe and dimensioned to be frictionally slidable along the inner wall of the syringe, and a spider element mounted in a fixed position on the plunger and having barb points engaged with the sleeve. When the plunger is first retracted, the spider element and sleeve travel toward the distal end of the syringe together with the plunger. When the plunger is depressed toward the injection end, the sleeve remains at the distal end, through frictional engagement with the inner wall, while the spider element travels toward the injection end with the plunger. If a second attempt is made to retract the plunger, the barbs of the spider element, now exposed to the inner wall in the absence of the sleeve, will engage the inner wall of the syringe and prevent a second retraction.

An alternative arrangement in the Hesse patent has the sleeve slidably supported on the plunger and engaged with the spider element having curved, spring-like prongs assembled at the distal end of the syringe. On the first retraction, the sleeve remains engaged with the spider element, and on the first depression, it is moved toward the injection end to expose the prongs of the spider element. An attempt to retract the plunger the second time will he prevented by engagement of the prongs with the plunger. Other devices, e.g. as shown in U.S. Patent 4,781,684 to Trenner, U.S. Patent 4,493,703 to Butterfield, U.S. Patent 4,391,272 to Staempfli, and U.S. Patent 4,367,738 to Legendre, provide for modifications to the plunger or syringe wall structure which allow only one-way movement of the plunger or which will lock or disable the plunger after a first depression.

Currently, a standard type of widely used disposable plastic syringe has a relatively simple construction of a cylindrical plastic wall formed with a closed injection end and an opposite open end, and a plastic plunger formed with a sealed plunger end, a knob end for depressing, and a stem in between the ends with an "X" shaped cross-section. The prior devices for preventing reuse of a syringe all involve structures which are built-in to the syringe at the time of manufacture. These devices cannot be retrofitted to the type of disposable plastic syringes which are already widely sold and commonly available. Moreover, their specialized construction would depart from that of the standard type of disposable plastic syringe, and would require retooling for manufacture and/or the marketing of a non-standard product.

### Summary of The Invention

Therefore, it is a principal object of the invention to provide a syringe including a locking element for preventing reuse of the syringe, said locking element being dimensioned so as to be insertable into the open end of the syringe between the stem of the plunger and the inner surface of the syringe wall. Specifically, it is intended that the locking element of the invention be capable of being retrofitted to or assembled with the standard type of disposable plastic syringes that are currently manufactured and widely distributed.

In accordance with the invention, a plastic syringe of the type having a wall elongated in a direction of a longitudinal axis thereof and provided with an inner surface of plastic material, a closed injection end at one end thereof and an open end at an opposite end thereof for movement of a plunger therein, includes a plunger with a plunger stem movable longitudinally along the axis of the syringe and a locking element dimensioned so as to be insertable into the open end of the syringe between the stem of the plunger and the inner surface of the syringe wall, wherein said locking element has at least a first set of barbed points for engaging said barbed element into the plastic material in a fixed position on said plunger stem, and a second set of barbed points which are angled relative to the syringe wall so as to be slidable along the inner surface of the syringe wall in a first direction of travel toward said injection end and to be fixedly engageable into the plastic material of said inner surface of said syringe wall in a second direction of travel away from said injection end, and wherein, upon a first retraction of said plunger stem in the second direction away from said injection end, said barbed element is held by said second set of barbed points in a fixed position engaged in said inner surface of said syringe wall while said first set of barbed points slide along said plunger stem, said first set of barbed points becoming engaged in said plunger stem when said retracted plunger piston contacts said barbed element, then upon a first depression of said plunger stem in said first direction toward said injection end, said barbed element is held by said first set of barbed points in a fixed position engaged in said plunger stem while said second set of barbed points slide along said inner surface of said syringe wall, and then upon a second attempted retraction of said plunger stem in said second direction away from said injection end, said first set of barbed points remain fixedly engaged in said plunger stem and said second set of barbed points become fixedly engaged in said inner surface of said syringe wall, thereby locking said plunger stem from the second retraction.

In one embodiment of the invention, the barbed element has an arched, spring portion provided with a first pair of barbed points which are spaced apart and facing in opposite directions along the axis of the syringe, and a second pair of barbed points spaced apart in a transverse direction and which are pointed at an angle away from said injection end. The first pair of barbed points does not fully engage in the plastic material of the plunger stem until it has been withdrawn once and depressed once toward the injection end. The plunger may not be withdrawn thereafter due to engagement of the second pair of barbed points in the plastic material of the syringe wall.

In another embodiment of the invention, the barbed element has a clip portion which snap-fits onto a rib portion of the plunger stem, and a second pair of barbed points spaced apart in a transverse direction and pointed at an angle away from the injection end so that the plunger may be depressed once toward the injection end, but may not be withdrawn thereafter. Preferably, the clip portion has a U-shaped cross section which fits over the rib portion of the plunger stem and a first pair of barbed points which are spaced apart in a transverse direction and facing inwardly on each respective side of the rib. The first pair of barbed points does not fully engage in the plastic material of the plunger rib until the plunger stem has been withdrawn once and depressed toward the injection end. The second pair of barbed points thereafter prevents the plunger from being withdrawn a second time.

Other objects, features, and advantages of the present invention will become apparent from the following detailed description of the preferred embodiments of the invention considered in conjunction with the drawings, as follows:

### Description of The Drawings

Figs. 1A, 1B, and 1C are side, top, and front views, respectively, of a spring-type barbed element for insertion in a standard plastic syringe in accordance with the invention;
Fig. 2 is a schematic view illustrating the construction of the barbed element of Figs. 1A-1C;
Figs. 3A, 3b, 3C, 3D, 3E, 3F, and 3G illustrate use of the barbed element of Figs. 1A-1C to prevent reuse of a standard plastic syringe;
Figs. 4A, 4B, and 4C are side, top, and front views, respectively, of a clip-type barbed element for insertion in a standard plastic syringe in accordance with the invention;
Fig. 5 is a schematic view illustrating the construction of the barbed element of Figs. 4A-4C; and
Figs. 6A, 6b, 6C, 6D, 6E, 6F, and 6G illustrate use of the barbed element of Figs. 4A-4C to prevent reuse of a standard plastic syringe.

### Description of The Preferred Embodiments

The invention in its broadest sense encompasses a locking device which can be inserted in a standard disposable plastic syringe for preventing its reuse. The locking device operates by the principle of a first portion having barbed points which engage in the plastic material of the syringe for holding the device in a fixed position on either of the plunger stem or the inner wall surface of the syringe after the plunger stem has been once withdrawn (for loading) and depressed, and a second portion having barbed points which engage in the opposing surface in order to prevent the plunger stem from being withdrawn a second time. In the following description, spring-type and clip-type embodiments are illustrated for use with standard plastic syringes. However, it is to be understood that the invention is not limited to these embodiments or types of syringes alone.

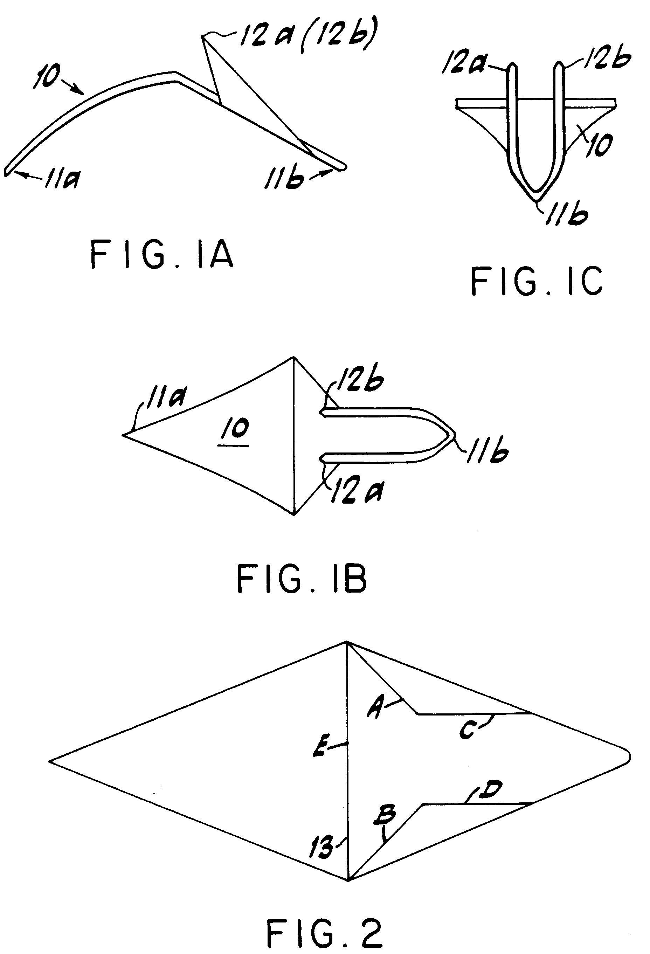

Referring to Figs. 1A-1C, a spring-type barbed element for insertion in a standard plastic syringe has a curved or arched body portion 10 extending in the longitudinal direction, which is also the direction of the plunger stem axis. The body portion 10 has a first pair of barbed points 11a, 11b spaced apart at opposite ends of the length thereof. The point 11a is intended to face away from the injection end of the syringe, as described in further detail below, and is shaped as a sharp point. The point 11b is intended to face toward the injection end and has the shape of a pointed scoop. A second pair of barbed points 12a, 12b extend outwardly from the body portion 10 and are inclined at an angle away from the injection end of the syringe. The points 12a, 12b are spaced apart in a transverse direction (perpendicular to the longitudinal direction) for lateral holding stability. The dimensions and angles shown in the drawings are given corresponding to the dimensions (inner bore of syringe, cross-section of plunger, etc.) of a standard plastic syringe sold under the trademark "B-D" of 5 cc. volume.

In Fig. 2, the steps for fabricating the spring-type barbed element from a single blank of metal are shown. A blank of sheet metal or other pliable metal may be used. Slits A and B are cut in the blank and are bent upward by bending up 90 degrees at bend lines C and D. The body portion 10 is formed by bending the opposite section down about 40 degrees along bend line E. The barbed insert is therefore very simple to fabricate and low cost.

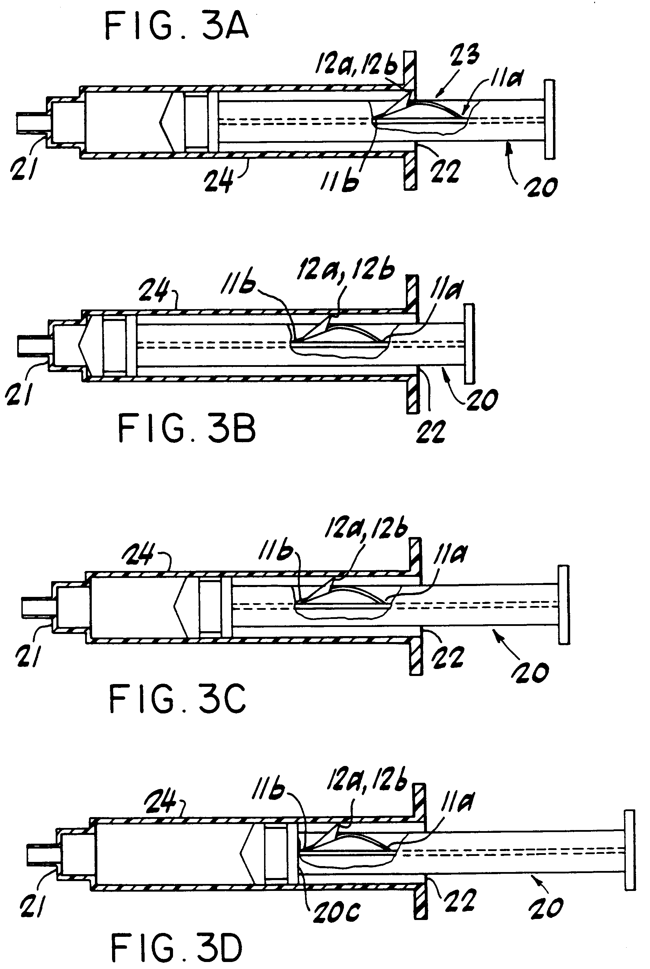

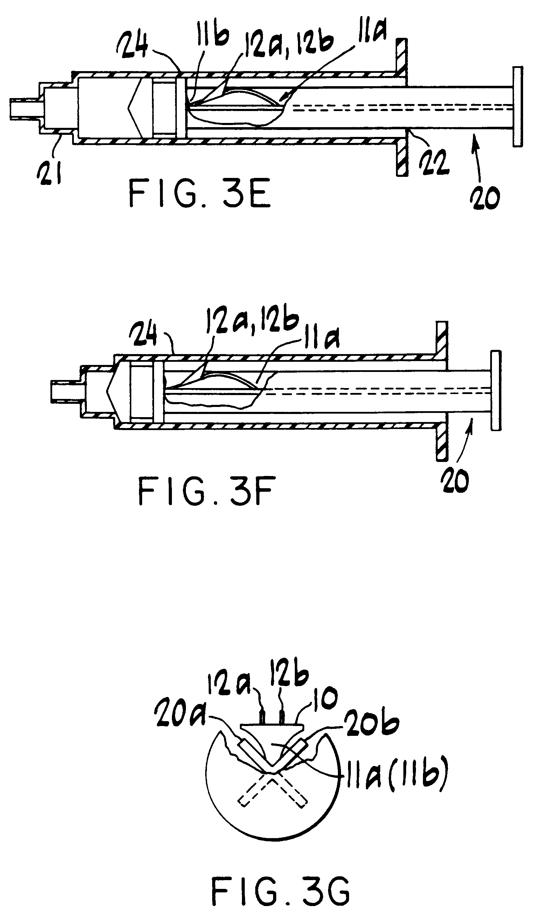

In Figs. 3A to 3G, the insertion and use of the spring-type barbed element insert in a "B-D" type plastic syringe is illustrated. In Fig. 3A, the plunger 20 is partially withdrawn and the barbed element is inserted in the open end 22 of the syringe between two ribs 20a, 20b of the plunger stem. As shown more clearly in Fig. 3G, the plunger stem has four ribs in an X-shaped cross-section, and the barbed element is fitted between two of the ribs 20a, 20b with the point 11a facing toward the open end 22, and the point 11b facing toward the injection end 21. A slight downward force is applied on the arched portion of the barbed element, as indicated by the arrow 23, so that the element is inserted between the inner surface of the syringe wall 24 and the plunger stem and held by a light spring force. The sharp barbed point 11a catches in the plastic material of the plunger stem.

In Fig. 3B, the plunger may be depressed into the syringe for packaging or in preparation for later use. The sharp barb points 12a, 12b catch in the plastic material of the syringe walls, so that the barbed element remains at the position shown. When the plunger is retracted for loading of fluid in the volume of the syringe walls, the barbed element remains at the catch position, while the scoop-shaped point 11b rides over the retracting plunger stem. As the sharp point 11a is facing in the same direction of plunger movement, it also rides over the plunger, thus allowing it to be withdrawn. At Fig. 3D, the plunger is fully withdrawn, and the scoop-shaped point 11b contacts the injection end 20c of the plunger and prevents further retraction. The spring action of the arched body of the barbed element combined with the force of contact with the plunger end 20a causes positive engagement of the points 11a, 11b in the plastic material of the plunger.

Upon depressing the plunger in an injection movement toward the injection end 21, as shown in Fig. 3E, the barbed element is locked in position on the plunger stem by points 11a, 11b, while the points 12a, 12b ride over the inner surface of the syringe walls due to their being inclined in the direction away from the injection end. When the plunger has been fully depressed, in Fig. 3F, the barbed element is located at the injection end of the plunger and is both locked in position on the plunger stem by the points 11a, 11b, as well as locked from a further retraction movement by the points 12a, 12b.

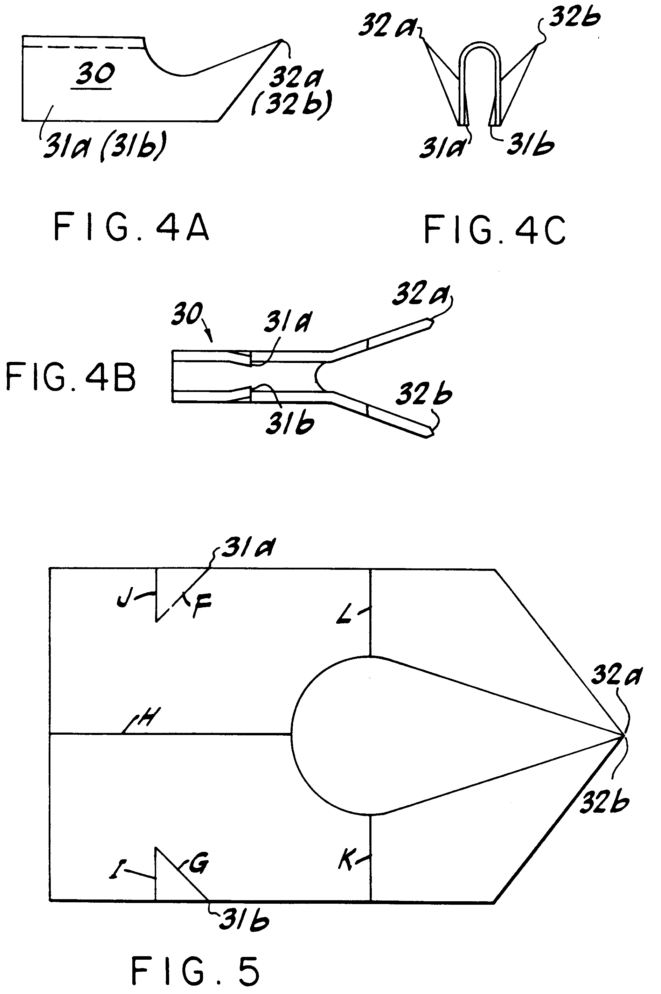

Referring to Figs. 4A-4C, a clip-type barbed element has a body portion 30 extending in the longitudinal direction which is formed with a U-shaped channel or recess for fitting onto one rib of the X-ribbed plunger stem. The body portion 30 has a first pair of barbed points 31a, 31b spaced apart and facing inwardly on opposite sides of the U-shaped channel of the body portion. A second pair of barbed points 32a, 32b extend outwardly from and above the body portion 30 and are inclined at an angle away from the injection end of the syringe. The points 32a, 32b are spaced apart in the transverse direction for lateral stability. The preferred dimensions and angles shown in the drawings are given for the example of a standard plastic syringe sold under the trademark "Monoject" of 3 cc. volume.

In Fig. 5, the steps for fabricating the clip-type barbed element from a single blank of metal are shown. Slits F and G are cut in the blank and are bent slightly inward along the bend lines I and J to form the points 31a, 31b. The body portion 10 is formed by bending the opposing sides curvedly along longitudinal bend line H. The barbed points 32a, 32b are formed by cutting out the oval shaped portion and separating the tips.

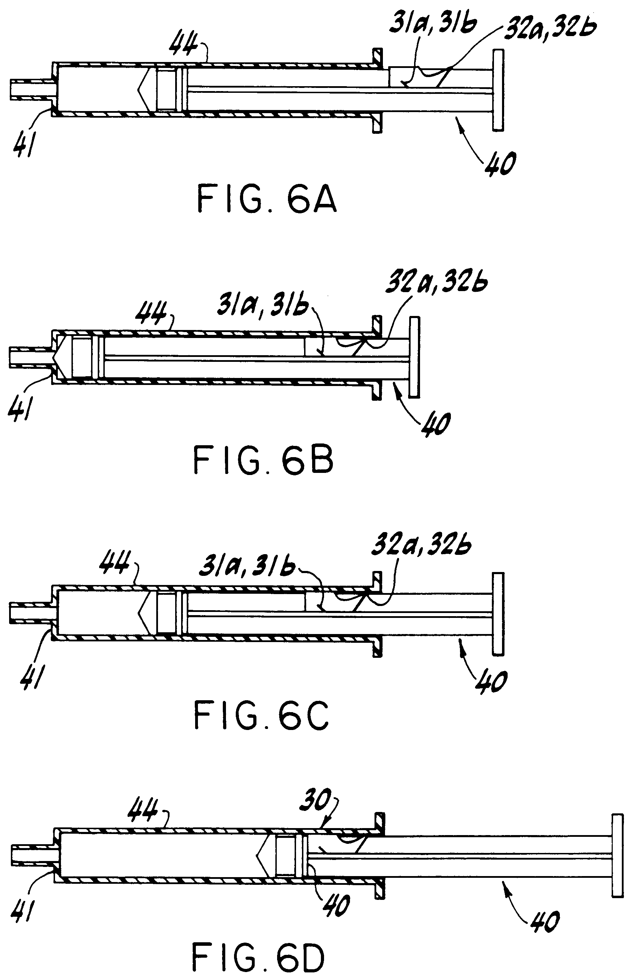

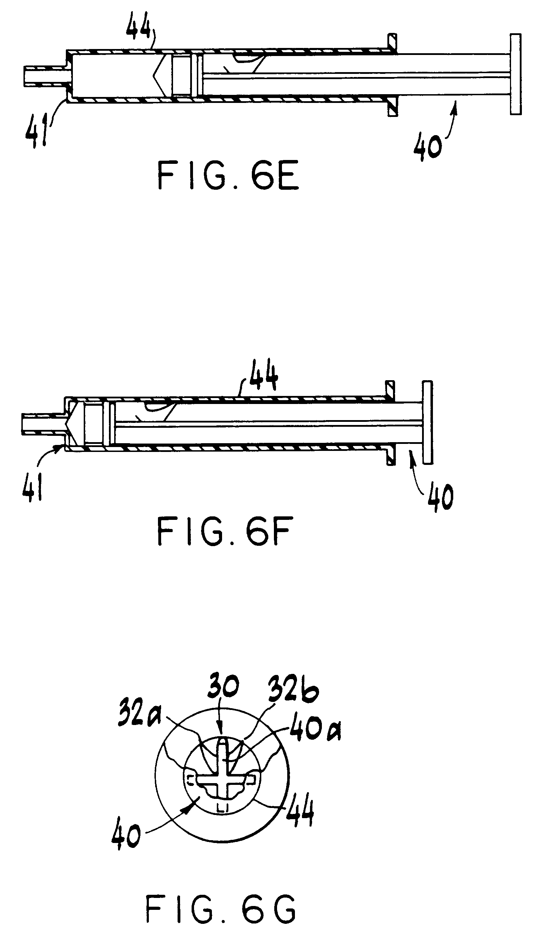

In Figs. 6A to 6G, the insertion and use of the clip-type barbed element insert in a "Monoject" type-plastic syringe is illustrated. In Fig. 6A, the plunger 40 is partially withdrawn and the barbed element is loaded by snap-fitting the U-shaped channel onto one rib 40a of the plunger stem, as shown more clearly in Fig. 6G, with the both sets of points 31a, 31b and 32a, 32b facing away from the injection end 41 of the syringe. The barbed points 31a, 31b catch in the plastic material of the plunger stem rib to hold the element in position thereon.

In Fig. 6B, the plunger is depressed into the syringe and the barbed element is held in position by the points 32a, 32b catching in the plastic material of the syringe walls. When the plunger is retracted for loading of fluid in the volume of the syringe walls, the barbed element is held at the catch position by the points 32a, 32b, while the points 31a, 31b ride over the retracting plunger stem. At Fig. 6D, the plunger is fully withdrawn, and the back end of the element contacts the injection end 40a of the plunger and prevents further retraction. The force of contact with the plunger end 40a drives the points 31a, 31b into deeper engagement in the plastic material to lock it into position on the plunger stem.

Upon depressing the plunger in an injection movement toward the injection end 41, as shown in Fig. 6E, the barbed element is locked in position on the plunger stem by points 31a, 31b, while the points 32a, 32b ride over the inner surface of the syringe walls due to their being inclined in the direction away from the injection end. When the plunger has been fully depressed, in Fig. 6F, the barbed element is located at the injection end of the plunger and is both locked in position on the plunger stem by the points 31a, 31b, as well as locked from a further retraction movement by the points 32a, 32b.

The invention is particularly advantageous in that the barbed element can be fabricated readily and inexpensively, and can be retrofitted to or assembled with the standard types of disposable plastic syringes that are currently in wide use. The arrangement of barbed points allow a first retraction of the plunger for loading the injection fluid, and positively engage in the plastic walls of the syringe to prevent a reuse of the syringe. Thus, the invention can be immediately adopted for use in fighting the spread of blood-transmitted diseases, such as AIDS.

Numerous modifications and variations are of course possible in light of the principles of the invention disclosed above. For example, the orientation and arrangement of the barbed points and the holding structure of the body portion of the barbed element may be modified readily. All such modifications and variations are intended to be included within the scope of the invention, as defined in the following claims.

## Claims

1. A non-reusable syringe comprising a syringe wall (24, 44) elongated in the direction of a longitudinal axis thereof and provided with an inner surface of plastic material, a closed injection end (21, 41) at one end thereof, and an open end (22, 42) at an opposite end thereof for movement of a plunger therein, a plunger (20, 40) having a piston on the end of its plunger stem movable along the longitudinal axis of the syringe, and a locking element for preventing reuse of the syringe, said locking element being dimensioned so as to be insertable into the open end of the syringe between the stem of the plunger and the inner surface of the syringe wall, said locking element having a pair of barbed points which are spaced apart at opposite ends of the element, characterized in that said locking element has a first set of barbed points (11, 31) for engaging said barbed element into the plastic material in a fixed position on said plunger stem, and a second set of barbed points (12, 32) which are angled relative to the syringe wall so as to be slidable along the inner surface of the syringe wall in a first direction of travel toward said injection end and to be fixedly engageable into the plastic material of said inner surface of said syringe wall in a second direction of travel away from said injection end, wherein, upon a first retraction of said plunger stem in the second direction away from said injection end, said barbed element is held by said second set of barbed points (12, 32) in a fixed position engaged in said inner surface of said syringe wall while said first set of barbed points (11, 31) slide along said plunger stem, said first set of barbed points becoming engaged in said plunger stem when said retracted plunger piston contacts said barbed element, then upon a first depression of said plunger stem in said first direction toward said injection end, said barbed element is held by said first set of barbed points (11, 31) in a fixed position engaged in said plunger stem while said second set of barbed points (12, 32) slide along said inner surface of said syringe wall, and then upon a second attempted retraction of said plunger stem in said second direction away from said injection end, said first set of barbed points (11, 31) remain fixedly engaged in said plunger stem and said second set of barbed points (12, 32) become fixedly engaged in said inner surface of said syringe wall, thereby locking said plunger stem from the second retraction.

2. A non-reusable syringe according to Claim 1, wherein said barbed element is a unitary element stamped and formed from a single plate of metal.

3. A non-reusable syringe as set forth in Claim 1 or 2, characterized in that said locking element is formed with a spring-type body portion (10) having said first set of barbed points (11) formed as a pair of barbed points which are spaced apart at opposite ends of said body portion and pointed in opposite directions along the longitudinal axis of said syringe at an angle to said plunger stem, said spring-type body portion being dimensioned and configured so as to be held by a slight spring force between said plunger stem and said inner surface of said syringe wall when said barbed element is inserterd into said syringe, and said second set of barbed points (12) are a pair of barbed points spaced apart in a transverse direction perpendicular to the longitudinal axis of said syringe and pointed at an angle toward the second direction away from said injection end of said syringe.

4. A non-reusable syringe according to Claim 1 or 2, characterized in that said locking element is formed with a clip-type body portion (30) having a U-shaped channel for fitting onto a longitudinal rib portion of said plunger stem, and said first set of barbed points (31) is formed as a pair of barbed points on said clip-type body portion which are spaced apart in a transverse direction perpendicular to the longitudinal axis of the syringe and pointed inwardly toward each other from opposite sides of said rib portion at an angle to said plunger stem in the second direction away from the injection end of said syringe, said clip-type body portion being dimensioned and configured so as to be held by a slight spring force on said plunger stem when said barbed element is inserted into said syringe, and said second set of barbed points (32) are a pair of barbed points spaced apart in a transverse direction perpendicular to the longitudinal axis of said syringe and pointed at an angle toward the second direction away from said injection end of said syringe.

5. A locking element as set forth in any of the preceding claims for insertion into the open end of a disposable syringe between the stem of the plunger and the inner surface of the syringe wall for preventing reuse of the syringe.

## Patentansprüche

1. Nicht-wiederverwendbare Spritze mit einer in Richtung deren Längsachse verlängerten Spritzenwand (24, 44) und ausgestattet mit einer Innenfläche aus Plastikmaterial, einem geschlossenen Spritzenende (21, 41) an ihrem einen Ende und einem offenen Ende (22, 42) an ihrem gegenüberliegenden Ende zur Bewegung eines Kolbens in ihr, einem Kolben (20, 40), der ein Druckkolbenelement am Ende seines Kolbenstabes hat, der entlang der Längsachse der Spritze beweglich ist, und einem Verschlußelement zum Vermeiden einer Wiederverwendung der Spritze, wobei das Verschlußelement so dimensioniert ist, daß es in das offene Ende der Spritze zwischen dem Stab des Kolbens und der Innenfläche der Spritzenwand einsetzbar ist, wobei das Verschlußelement ein Paar Wiederhaken hat, die mit Zwischenraum voneinander an gegenüberliegenden Enden des Elements angeordnet sind, dadurch gekennzeichnet, daß das Verschlußelement einen ersten Satz Wiederhaken (11, 31) hat, um das Wiederhakenelement in das Plastikmaterial in einer festen Position auf dem Kolbenstab eingreifen zu lassen, und einen zweiten Satz Wiederhaken (12, 32), die bezüglich der Spritzenwand gewinkelt angeordnet sind, um entlang der Innenfläche der Spritzenwand in einer ersten Verschiebungsrichtung zu dem Spritzenende hin gleiten zu können, und um in einer zweiten Verschiebungsrichtung weg von dem Spritzenende fest in das Plastikmaterial der Innenfläche der Spritzenwand eingreifen zu können, wobei bei einem ersten Einziehen das Kolbenstabes in die zweite Richtung weg von dem Spritzenende das Wiederhakenelement von dem zweiten Satz Wiederhaken (12, 32) in einer festen Position in die Innenfläche der Spritzenwand eingegriffen gehalten wird, während der erste Satz Wiederhaken (11, 31) entlang des Kolbenstabes gleitet, wobei der erste Satz Wiederhaken in den Kolbenstab eingreift, wenn das eingezogene Kolben-Druckkolbenelement das Wiederhakenelement berührt, und dann bei einem ersten Runterdrücken des Kolbenstabes in die erste Richtung zu dem Spritzenende hin das Wiederhakenelement von dem ersten Satz Wiederhaken (11, 31) in einer festen Position in den Kolbenstab eingegriffen gehalten wird, während der zweite Satz Wiederhaken (12, 32) entlang der Innefläche der Spritzenwand gleitet, und dann bei einem zweiten versuchten Einziehen des Kolbenstabes in die zweite Richtung weg von dem Spritzenende, der erste Satz Wiederhaken (11, 31) fest eingegriffen in den Kolbenstab bleibt und der zweite Satz Wiederhaken (12, 32) fest in die Innenfläche der Spritzenwand eingreift, wodurch dem Kolbenstab ein zweites Einziehen versperrt wird.

2. Nicht-wiedervendbare Spritze gemäß Anspruch 1, bei der das Wiederhakenelement ein aus einer einzigen Metallplatte gestanztes und gebildetets Einheiten-Element ist.

3. Nicht-wiedervendbare Spritze gemäß Anspruch 1 oder 2, dadurch gekennzeichnet, daß das Verschlußelement mit einem Fedorkörperbereich (10) gebildet ist, der den ersten Satz Wiederhaken (11) als ein Paar Wiederhaken hat, die mit Zwischenraum voneinander an gegenüberliegenden Enden des Körperbereichs angeordnet und in entgegengesetzte Richtungen entlang der Längsachse der Spritze in einem Winkel zu dem Kolbenstab spitzzulaufend sind, wobei der Federkörperbereich so dimensioniert und konfiguriert ist, daß er von einer leichten Federkraft zwischen dem Kolbenstab und der Innenfläche der Spritzenwand gehalten wird, wenn das Wiederhakenelement in die Spritze eingesetzt wird, und der zweite Satz Wiederhaken (12) ein Paar Wiederhaken ist, die mit Zwischenraum voneinander in einer Querrichtung senkrecht zur Längsachse der Spritze angeordnet und in einem Winkel in die zweite Richtung, weg von dem Spritzenende der Spritze spitzzulaufend sind.

4. Nicht-wiederverwendbare Spritze gemäß Anspruch 1 oder 2, dadurch gekennzeichnet, daß das Verschlußelement mit einem Klemmkörperbereich (30) gebildet ist, der einen Kanal in U-Form hat, um auf einen Längsrippenbereich des Kolbenstabes zu passen, und der erste Satz Wiederhaken (31) als ein Paar Wiederhaken auf dem Klemmkörperbereich gebildet ist, die mit Zwischenraum voneinander in einer Querrichtung senkrecht zu der Längsachse der Spritze angeordnet und nach innen zueinander von entgegengesetzten Seiten des Klemmkörperbereichs in einem Winkel zu dem Kolbenstab in der zweiten Richtung, weg von dem Spritzenende der Spritze spitzzulaufend sind, wobei der Klemmkörperbereich so dimensioniert und konfiguriert ist, daß er durch eine leichte Federkraft auf dem Kolbenstab gehalten wird, wenn das Wiederhakenelement in die Spritze eingesetzt wird, und der zweite Satz Wiederhaken (32) ein Paar Wiederhaken ist, die mit Zwischenraum voneinander in einer Querrichtung senkrecht zu der Längsachse der Spritze angeordnet und in einem Winkel in die zweite Richtung, weg von dem Spritzenende der Spritze spitzzulaufend sind.

5. Verschlußelement gemäß irgendeinem der vorherigen Ansprüche zum Einsetzen in das offene Ende einer Einwegspritze zwischen den Stab des Kolbens und der Innenfläche der Spritzenwand, um eine Wiederverwendung der Spritze zu verhindern.

## Revendications

1. Une seringue non-réutilisable comprenant une paroi (24, 44) allongée dans la direction de son axe longitudinal et munie d'une surface intérieure en matériau plastique, une extrémité d'injection fermée (21, 41) à une extrémité et une extrémité ouverte (22, 42) à l'extrémité opposée pour le déplacement d'un plongeur, un plongeur (20, 40) ayant un piston à l'extrémité de sa tige mobile suivant l'axe longitudinal de la seringue, et un élément bloquant pour empêcher la réutilisation de la seringue, ledit élément bloquant étant dimensionné afin d'être insérable dans l'extrémité ouverte de la seringue entre la tige du plongeur et la surface intérieure de la paroi de la seringue, ledit élément bloquant ayant une paire de pointes qui sont disposées aux extrémités opposées de l'élément, caractérisée en ce que ledit élément bloquant a un premier ensemble de pointes (11, 31) pour insérer ledit élément bloquant dans le matériau plastique dans une position fixe sur ladite tige du plongeur, et un second ensemble de pointes (12, 32) qui forment un angle avec la paroi de la seringue afin de pouvoir glisser le long de la surface interne de la paroi de la seringue dans une première direction de déplacement vers ladite extrémité d'injection et de s'insérer fixement dans le matériau plastique de la surface intérieure de la paroi de la seringue dans une seconde direction de déplacement s'éloignant de ladite extrémité d'injection de manière que lors d'un premier retrait de la tige du plongeur dans la seconde direction s'éloignant de l'extrémité d'injection, ledit élément soit maintenu par ledit second ensemble de pointes (12, 32) dans une position fixe en étant inséré dans ladite surface intérieure de la paroi de la seringue alors que ledit premier ensemble de pointes (11, 31) glisse le long de la tige du plongeur, ledit premier ensemble de pointes s'insérant dans la tige du plongeur lorsque le piston plongeur rétracté entre en contact avec ledit élément, que ensuite, lors d'un premier enfoncement de ladite tige du plongeur dans ladite première direction vers ladite extrémité d'injection, ledit élément bloquant soit maintenu par le premier ensemble de pointes (11, 31) dans une position fixe en étant inséré dans la tige du plongeur pendant que le second ensemble de pointes (12, 32) glisse le long de la surface intérieure de la paroi de la seringue, et qu'enfin, lors d'une seconde tentative de retrait de la tige du plongeur dans ladite seconde direction s'éloignant de ladite extrémité d'injection, le premier ensemble de pointes (11, 31) reste fixement inséré dans la tige du plongeur et ledit second ensemble de pointes (12, 32) devient fixement inséré dans la surface intérieure de la paroi de la seringue, de manière à bloquer ladite tige de plongeur pour en empêcher un second retrait.

2. Une seringue non-réutilisable selon la revendication 1, dans laquelle ledit élément bloquant est un élément unitaire estampé et formé d'une seule pièce de métal.

3. Une seringue non-réutilisable selon la revendication 1 ou 2, caractérisée en ce que ledit élément bloquant est formé d'un corps du type à ressort (10) ayant ledit premier ensemble de pointes (11) formés par une paire de pointes qui sont disposées aux extrémités opposées dudit corps et dirigées dans des directions opposées le long de l'axe longitudinal de la seringue en formant un angle avec la tige du plongeur, ledit corps du type à ressort étant dimensionné et configuré de manière à être maintenu par une légère force élastique entre la tige du plongeur et ladite surface intérieure de la paroi de la seringue lorsque ledit élément bloquant se trouve inséré dans la seringue, et ledit second ensemble de pointes (12) est une paire de pointes disposées dans une direction transversale perpendiculaire à l'axe longitudinal de la seringue en formant un angle dans la seconde direction s'éloignant de ladite extrémité d'injectin de la seringue.

4. Une seringue non-réutilisable selon la revendication 1 ou 2, caractérisée en ce que ledit élément bloquant est formé par un corps du type à griffes (30) ayant un canal en forme de U pour s'adapter sur une partie nervurée longitudinale de la tige du plongeur, et ledit premier ensemble de pointes (31) est formé d'une paire de pointes disposées sur le corps du type à griffes en étant écartées l'une de l'autre dans une direction transversale perpendiculaire à l'axe longitudinal de la seringue et étant dirigées vers l'intérieur l'une vers l'autre à partir des côtés opposés dudit corps en formant un angle avec la tige du plongeur dans la seconde direction s'éloignant de l'extrémité d'injection de la seringue, ledit corps du type à griffes étant dimensionné et configuré de manière à être maintenu par une légère force élastique sur la tige du plongeur lorsque ledit élément bloquant se trouve inséré dans la seringue, et ledit second ensemble de pointes (32) est une paire de pointes disposées dans une direction transversale perpendiculaire à l'axe longitudinal de la seringue en formant un angle dans la seconde direction s'éloignant de ladite extrémité d'injection de la seringue.

5. Un élément bloquant selon l'une quelconque des revendications précédentes pour l'insertion dans l'extrémité ouverte d'une seringue jetable entre la tige du plongeur et la surface intérieure de la paroi de la seringue pour empêcher la réutilisation de la seringue.
